(19) Europäisches Patentamt / European Patent Office / Office européen des brevets

(11) **EP 4 213 247 A1**

(12) **EUROPEAN PATENT APPLICATION**
published in accordance with Art. 153(4) EPC

(43) Date of publication:
**19.07.2023 Bulletin 2023/29**

(21) Application number: **21866142.9**

(22) Date of filing: **07.09.2021**

(51) International Patent Classification (IPC):
*H01M 4/58* (2010.01)    *B01J 23/00* (2006.01)
*C01G 31/02* (2006.01)    *C25B 1/04* (2021.01)

(52) Cooperative Patent Classification (CPC):
Y02E 60/10; Y02E 60/36

(86) International application number:
**PCT/ES2021/070649**

(87) International publication number:
**WO 2022/053730 (17.03.2022 Gazette 2022/11)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **08.09.2020 ES 202030912**

(71) Applicant: **Universidade de Santiago de
Compostela
15782 Santiago de Compostela (ES)**

(72) Inventors:
• **GIMENEZ LOPEZ, Maria del Carmen
15782 Santiago de Compostela (ES)**
• **QUIROS DIEZ, Eugenia Pilar
15782 Santiago de Compostela (ES)**
• **GUILLEN SOLER, Melanie
15782 Santiago de Compostela (ES)**

(74) Representative: **Patentree
Edificio Net
Rua de Salazares, 842
4149-002 Porto (PT)**

(54) **COMPOUND FOR BATTERIES**

(57) The present invention relates to a compound for splitting water by means of an electrolysis process. More specifically, the compound is useful for producing hydrogen and for producing oxygen. The invention also relates to an electrode, a battery and the methods for preparing the electrode.

EP 4 213 247 A1

**Description**

**TECHNICAL FIELD**

The present invention relates to a compound for splitting water by means of an electrolysis process. More specifically, the compound is useful for producing hydrogen and for producing oxygen. The invention also relates to an electrode, the methods for preparing the electrode, a battery (or electrochemical cell) the method for preparing the compound and uses thereof.

**BACKGROUND**

The development of highly active, versatile and durable electrocatalytic materials for the production of clean and sustainable energy is at the forefront of the technological challenges facing humanity.
Electrochemical technologies largely depend on rare and endangered chemical elements, such as platinum, iridium or ruthenium, whose scarcity is creating a bottleneck for future technological progress. Therefore, the use of more abundant and greener sources is highly desirable.
In addition, these elements often only work well for one type of reaction. For example, the best-known catalyst for water reduction is platinum, whereas iridium and ruthenium are used for water oxidation.
Since the surface requirements of the catalyst during water reduction and oxidation are completely different, the development of bifunctional catalysts for water splitting, which in turn should be compatible with operation under acidic conditions and prevent corrosion, is therefore extremely difficult.
Polyoxometalate clusters have been applied as electrocatalysts for both water reduction and oxidation, although they are mainly restricted to use at neutral and basic pH, especially in the water oxidation reaction.
Therefore, it is still necessary to design new bifunctional electrocatalysts that can be used at acidic pH, thus allowing their use at industrial scale, preferably with the use of proton exchange membranes that present a high current density together with the possibility of producing high purity hydrogen gas.

**BRIEF DESCRIPTION OF THE INVENTION**

The authors of the present invention have designed a new polyoxometalate (POM)-based compound capable of performing kinetically fast water oxidation or reduction under strong acidic conditions, for example at pH below 1, when on an electrode. In addition, it allows electrolysis under acidic conditions, being much more stable than other electrodes.
Therefore, in a first aspect, the invention refers to a compound (I) comprising

a) a polyoxometalate,
b) at least one ligand comprising a head which is capable of attracting protons and a tail which in an acidic medium has at least one positive charge,
c) a positively charged counterion,
d) at least one water molecule.

In a second aspect, the invention refers to an electrode comprising a compound (I) such as described above.
In a third aspect, the invention refers to the preparation of the electrodes comprising a compound (I) as described above.
In a fourth aspect, the invention refers to a battery comprising an electrode such as described in the second aspect of the invention.
In a fifth aspect, the invention refers to a method for preparing the compound of the first aspect of the invention.
Another aspect of the invention refers to the use.

**DESCRIPTION OF THE DRAWINGS**

Figure 1 shows SEM images at 20 kV of the compound prepared in example 1.

Figure 2 shows the (a) Raman and (b) FT-IR spectra of compound (1) prepared in example 1.

Figure 3 shows the comparison of the measurements of cyclic voltammograms at 50 mV/s under nitrogen atmosphere using sulphuric acid in the electrolyte, of: a) 1/BC prepared in example 2.2 and BC; and b) 1/CNT prepared in example 2.2 and CNT.

Figure 4 shows the comparison of the oxygen evolution reaction (OER) behaviour of 1/CNT prepared in example 2.2, CNT and Ir/C.

Figure 5 shows the comparison of the oxygen evolution reaction (OER) behaviour of the electrodes 1/CNT; $(TRIS)_6[V_{10}O_{28}]$/CNT; and $Na_6[V_{10}O_{28}]$/CNT, highlighting the effect of the ligand and the positively charged counterion.

Figure 6 shows an HR-TEM image of 1@CNT prepared in example 4.1.

Figure 7 shows the comparison of the hydrogen evolution reaction (HER) behaviour of 1/CNT prepared in example 2.2, 1@CNT prepared in example 4.2, and CNT. The black arrow shows the change of electrochemical behaviour.

Figure 8 shows the comparison of the hydrogen evolution reaction (HER) behaviour of the electrodes 1@CNT; $(TRIS)_6[V_{10}O_{28}]$@CNT; and $Na_6[V_{10}O_{28}]$@CNT, highlighting the effect of the ligand and the positively charged counterion.

Figure 9 shows the comparison of chronoamperometry measurements of 1@CNT//1/CNT and Pt/C//Ir/C in a two-electrode configuration under acidic conditions (pH < 0.5); and the photograph of the typical water splitting cell (two-electrode system) in which 1/CNT is used on the positive electrode and 1@CNT on the negative electrode, in 1 M $H_2SO_4$, applying a potential of 3.1 V for 120 s.

## DETAILED DESCRIPTION OF THE INVENTION

In a first aspect, the invention refers to a compound (I) comprising

a) a polyoxometalate,
b) at least one ligand comprising a head which is capable of attracting protons and a tail which in an acidic medium has at least one positive charge,
c) a positively charged counterion,
d) at least one water molecule.

In a particular embodiment, the metal of the polyoxymethalate in compound (I) is selected from vanadium, tungsten, molybdenum, niobium, tantalum, zirconium and titanium.

In a particular embodiment, the positively charged counterion in compound (I) is selected from sodium, potassium and lithium.

In a particular embodiment, the ligand head in compound (I) is a linear or branched, cyclic or non-cyclic hydrocarbon chain having between 1 to 12 carbon atoms, with more than 2 hydroxyl groups.

In a particular embodiment, the ligand head in compound (I) is a group of formula $(AlqOH)_3Alq$, wherein each Alq can be the same or different and is independently selected from a linear or branched, cyclic or non-cyclic hydrocarbon chain having between 1 to 12 carbon atoms.

In a particular embodiment, each Alq in compound (I) can be the same or different and is independently selected from a linear or branched non-cyclic hydrocarbon chain having between 1 to 4 carbon atoms.

In a particular embodiment, the tail in compound (I) is selected from an amino group and phosphonium.

In a particular embodiment, the ligand in compound (I) has the formula tris(hydroxyalkyl($C_1$-$C_4$)aminoalkyl($C_1$-$C_4$).

In a particular embodiment, compound (I) is $Z_n(H_2O)_m[(AlqOH)_3AlqNH_2]_l[M_XO_Y]$,

wherein Z is an alkali metal selected from sodium, potassium and lithium,
each Alq can be the same or different and is independently selected from a linear or branched, cyclic or non-cyclic hydrocarbon chain having between 1 to 12 carbon atoms, preferably is a $C_1$-$C_4$ alkyl,
M is a transition metal selected from vanadium, tungsten, molybdenum, niobium, tantalum, zirconium and titanium,
n has a value between 2 and 10,
m has a value between 0 and 16,
l has a value of 1, 2, 3 or 4,
x has a value between 6 and 36,

y has a value between 16 and 112.

In a particular embodiment, the compound of formula (I) is $Na_4 (H_2O)_{12}[(CH_2OH)_3CNH_3]_2[V_{10}O_{28}].4H_2O$.

In a second aspect, the invention refers to an electrode comprising compound (I) as described above.

In a particular embodiment, the electrode further comprises a carbon source. In a more particular embodiment, the carbon source is selected from a carbon nanotube, black carbon and a carbon nanofibre, preferably a multi-walled nanotube.

The interaction of compound (I) with the carbon source influences the usefulness of the electrode of the invention. Thus, when the compound (I) is simply physically mixed with the carbon source in the electrode of the invention, then the electrode is useful for the production of oxygen, as demonstrated in example 2. When the carbon source is a multi-walled nanotube, the best results in the production of oxygen are obtained, as demonstrated by the higher current density when compared to the current density when the carbon source is black carbon.

The hypothesis that can explain this reaction is that the cation is stabilised by the ligand and in this configuration the cation activates the oxygen atom of the water, and finally the oxidation of water takes place.

Therefore, in a particular embodiment, the invention relates to an electrode comprising a physical mixture of the compound of formula (I) and a carbon source. Preferably the carbon source is a carbon nanotube, more preferably a multi-walled nanotube. In another embodiment the invention relates to the use of this electrode for the production of oxygen or for the oxidation of water.

However, when compound (I) is assembled (assembly) or coating the surface of the carbon source, the electrode is useful for the production of hydrogen, as demonstrated in example 4.

The hypothesis that can explain the generation of hydrogen due to this interaction of compound (I) with the carbon source is that the ligand acts as a sponge, supporting polyoxometalate in the attraction of hydrogen atoms and finally the reduction of water takes place.

In a particular embodiment, the invention relates to an electrode comprising compound (I) coating the surface of a carbon source. In another embodiment the invention relates to the use of this electrode for the production of hydrogen or for the reduction of water.

In another embodiment, the invention relates to a method for preparing an electrode for the production of oxygen, comprising:

a) grinding a carbon source, preferably a carbon nanotube,
b) mixing the result of step a) with compound (I) of the invention until a homogeneous mixture is obtained, and then suspending it in an oil, and
c) spreading the suspension resulting from step b) on a support.

The grinding of step a) can be carried out in a mortar or similar equipment, and is intended to obtain particles of smaller size from the carbon source. The person skilled in the art may select different oils available in the art to prepare the suspension, such as a mineral oil.

In another embodiment, the invention relates to a method for preparing an electrode for the production of hydrogen, comprising:

a) dissolving compound (I) of the invention in water,
b) adding the solution of a) under sonication onto a carbon source, previously ground,
c) filtering,
d) resuspending the solid obtained in c), and
e) dispersing the mixture obtained in d) on a support.

In step b) of this process, the addition of the solution may be carried out dropwise or by means of an addition equipment in which the amount added over time can be controlled to ensure assembly of compound (I) on the carbon surface. The resuspension of the solid described in step d) can be carried out with materials generally employed for this purpose and known to the skilled person in the art, such as polymers, copolymers, oils, in some cases using also an alcoholic or glycolic solvent or mixtures thereof.

In a particular embodiment, the carbon source used in the methods of the invention is selected from carbon nanotubes, black carbon or carbon nanofibres.

In a fourth aspect, the invention refers to a battery comprising an electrode as described in the third aspect of the invention.

In a particular embodiment, the electrolyte of the battery of the invention has an acidic pH, preferably between 0.05 and 5.

As demonstrated in example 6, a battery prepared with electrodes formed by a compound of formula (I), wherein the electrolyte has a very acidic pH, for example below pH=1, is much more stable than a battery formed by electrocatalysts which are reference in the prior art, such as Pt / C and Ir / C. This constitutes a significant advantage of the electrodes of the invention, since they allow working for longer periods at a strong acidic pH.

In a fifth aspect the invention refers to the preparation of a compound (I), comprising:

a) dissolving a salt of a metal oxide in an aqueous solution, and
b) adding a ligand comprising a head which is capable of attracting protons and a tail which in an acidic medium has at least one positive charge.

Step b) of this process may require heating the mixture between 25°C and 100°C under stirring. The process may require a final filtration to remove by-products precipitating in the reaction medium, in order to obtain the final compound by evaporation of the water from the solution. This evaporation process can be carried out at atmospheric conditions or under reduced pressure.

The following examples serve to illustrate the invention and should not be considered a limitation thereof.

**Materials and Methods**

Sodium metavanadate (96%) and HCl (37%) were purchased from Alfa Aesar. Ultrapure (>99%) tris (hydroxymethyl) aminomethane (TRIS) was obtained from Molekula.

All these reagents and solvents were used as received. Carbon nanotubes (CNT), multi-walled nanotubes, were purchased from Pyrograf Products Inc., USA. XC-72 black carbon (BC) was purchased from FuelCellStore, mineral oil (Nujol) was purchased from Sigma Aldrich. Carbon supported Ir (Ir / C) (20% Ir) was obtained from Premeter co. Carbon supported Platinum (Pt / C) (20% Pt) and HISPEC 3000 was purchased from Alfa Aesar.

Infrared spectra were measured using a Bruker Alpha FTIR spectrometer with a platinum ATR module. Milling was carried out using a Retsch MM400 high-energy ball mill instrument.

Voltammetric measurements.

Electrochemical experiments were carried out in a typical three-electrode configuration with an AUTOLAB 302N electrochemical potentiostat using a glassy carbon electrode (GCE), a hydrogen electrode (RHE) and Pt wire as working, reference and counter electrode, respectively. Cyclic voltammetry was carried out in 1 M $H_2SO_4$ saturated with nitrogen at a scan rate of 50 mV/s between -0.5 V and 1.8 V.

Electrocatalytic measurements.

The electrocatalytic activity of the prepared catalyst electrodes was examined by polarisation curves using linear sweep voltammetry (LSV) at a scan rate of 5 mV/s conducted in a 1M $H_2SO_4$ solution. For the oxygen release reaction, the electrolyte was previously saturated with oxygen and the material was compared to commercial iridium on carbon (Ir / C), while for the hydrogen release reaction the electrolyte was saturated with hydrogen prior to the measurements, and the material was compared to commercial platinum on carbon (Pt / C) under the same conditions.

Oxygen production was detected with an Ocean Optics NeoFOX oxygen detection system equipped with a pre-calibrated FOXY probe inserted into the electrolyte.

Faradaic Efficiency (FE) was calculated using the following equation:

$$FE = experimental\ moles\ of\ O_2\ released\ (measured\ with\ NeoFOX)\ /\ theoretical\ moles\ of\ O_2$$

Where the theoretical moles of $O_2$ were calculated using the following equation:

$$Theoretical\ moles\ of\ O_2\ released = Q\ /\ nF$$

where $Q$ is the charge produced (Coulombs), $n$ is the number of electrons during the oxygen evolution reaction (4 electrons) and $F$ is the Faraday constant. The experimental moles of oxygen released during the oxygen evolution reaction were measured by NeoFox.

**Example 1. Preparation of $Na_4(H_2O)_{12}[(CH_2OH)_3CNH_3]_2[V_{10}O_{28}].4H_2O$ (1)**

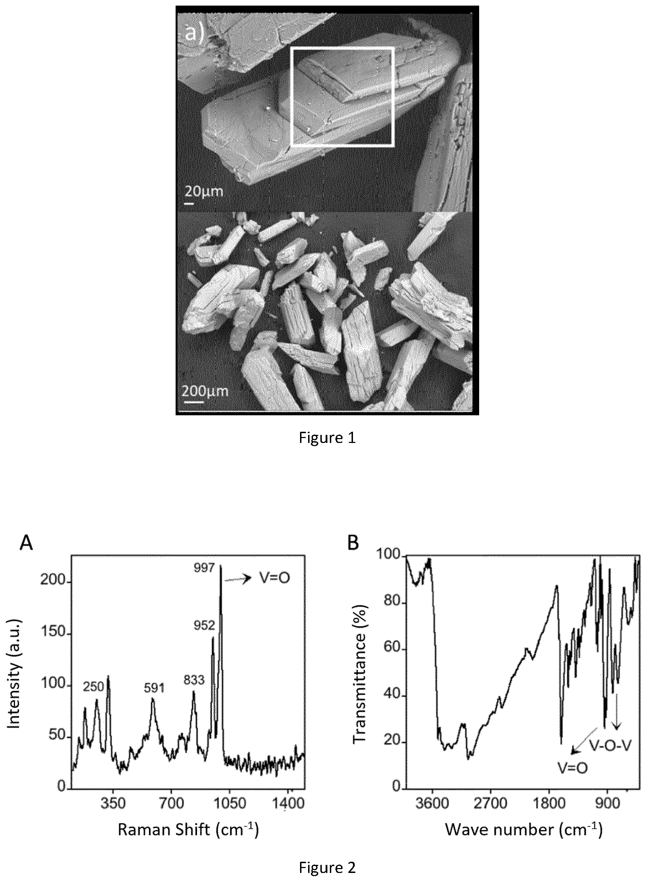

In a 100 mL round bottom flask, 2.51 g of NaVOs (20.6 mmol, 5 eq) were dissolved in 21 mL of water under stirring and heated to 85 °C. After dissolving $NaVO_3$, 6.18 mL (12.36 mmol, 3 eq) of an aqueous HCl solution (2 M) were added dropwise until a pH of 4 was reached. The initial yellow solution turned dark red after addition of the acid. Then, 0.5 g of TRIS [$(CH_2OH)_3CNH_2$] (4.12 mmol, 1 eq) were added to the solution and the reaction temperature was maintained at 85 °C for 6 hours with stirring. After that, the reaction was allowed to cool gradually to room temperature. The solution was filtered to remove the precipitate formed. The orange-red filtrate was allowed to evaporate slowly and after one day orange crystals of compound **1** were obtained and collected by filtration (10% yield of $Na_4(H_2O)_{12}[(CH_2OH)_3CNH_3]_2[V_{10}O_{28}].4H_2O$ (**1**)) (Figure 1).

The compound obtained was characterised by Raman (Figure 2a) and FT-IT (Figure 2b).

**Example 2. Preparation of an electrode for oxygen production (OER)**

2.1. Preparation of "short CNT". CNT were shortened by mechanical ball milling. In a typical experiment, 50 mg of CNT were placed in a stainless steel container (5 mL) with a stainless steel ball (10 mm diameter) and ground in air for 90 min at 10 Hz.

2.2. Preparation of the OER electrode. The carbon paste modified electrode (CPME) was prepared as follows. In a typical experiment, 22.5 mg of the carbon material to be used (BC or short CNT) and 2 mg of compound 1 obtained in example 1 were mixed in a mortar to obtain a homogeneous mixture, to which 50 μL of Nujol were added to form a suspension. It was then spread on a Glassy Carbon Electrode (GCE) with a surface area of 1 $cm^2$. The electrode was allowed to dry before electrochemical measurements.

The cyclic voltammogram (CV) of the CMPE obtained in acidic media showed that the observed changes in current with variations in the applied potential can only be assigned to **1,** as both the electrode additives used (i.e., mineral oil, CNT and BC) and the TRIS ligand show no redox process under the same conditions.

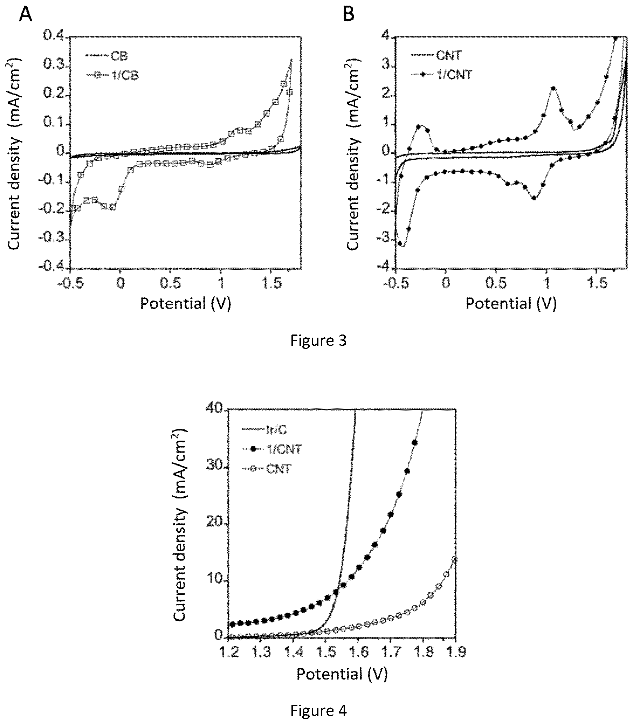

The cyclic voltammogram under acidic conditions (pH < 0.5) revealed current density values ten times higher for 1/CNT, when compared to 1/BC for the same amount of 1 (Figures 3a and 3b). While the CNT only shows a double-layer capacitance in the - 0.5 to 1.4 V potential region, 1/CNT exhibits reversible one-electron redox processes at -0.24, 1.05 and 1.2 V assigned to the V4+/V5+ pair of different vanadiums in the cluster. Moreover, 1/CNT exhibited an unexpected higher OER activity when compared to 1/BC and all other recently reported polyoxometalate-based electrocatalyst electrode materials, of which only very few are active at acidic pH (Table 1), which is quite remarkable. Surprisingly, the values obtained for the onset potential and the overpotential at 10 (mA / $cm^2$) are relatively small (1.45 and 1.58 V) as required for a highly active OER electrocatalyst electrode material. And these values are very close to the values obtained for the standard iridium (Ir / C) electrocatalyst (1.42 V and 1.48 V) used under the same conditions (Figure 4).

**Table 1.** Comparative summary of the performance of different POM-based electrocatalyst materials for OER.

| Material | Electrolyte | Onset potential (V) | Overpotential (V) at 10 (mA/cm$^2$) | Tafel slope (mV/dec) | Reference |
|---|---|---|---|---|---|
| 1/CNT | 1M H$_2$SO$_4$ | 1.45 | 1.58 | 370 | Invention |
| Ru$_4$Si$_2$-APS/NF | 0.2M Na$_2$PO$_4$ | 1.75 | 1.8 | 172 | [1] |
| PBA@POM | 1M KOH | 1.52 | 1.67 | 23.45 | [2] |
| 3a[Cs] | 50mM KPi buffer | 1.25 | - | 216 | [3] |
| 3b[Cs] | 50mM KPi buffer | 1.25 | - | 228 | [3] |

(continued)

| Material | Electrolyte | Onset potential (V) | Overpotential (V) at 10 (mA/cm$^2$) | Tafel slope (mV/dec) | Reference |
|---|---|---|---|---|---|
| Co$_4$POM | 0.1M Na$_3$PO$_4$ | 1.6 | - | 203 | [4] |

[1] Y. Ding, H. Li, and Y. Hou, Mater. Lett., 2018, 221, 264-266.
[2] Y. Wang, Y. Wang, L. Zhang, C.-S. Liu, and H. Pang, Chem. Asian J., 2019, 14, 2790 - 2795.
[3] M. Martín-Sabi, J. Soriano-López, R. S. Winter, J-J. Chen, L. Vilà-Nadal, D-L. Long, J. R. Galán-Mascarós, and L. Cronin, Nat. Catal., 2018, 1, 208-213.
[4] G. Y. Lee, I. Kim, J. Lim, M. Y. Yang, D. S. Choi, Y. Gu, Y. Oh, S. H. Kang, Y. S. Nam, and S. O. Kim, J. Mater. Chem. A., 2017, 5, 1941-1947.

In addition, an oxygen sensor was immersed in the electrolyte to confirm oxygen production when performing a linear sweep voltammogram from 1.0V to 1.9V. Indeed, there was an increase in density at 1.44V after approximately 45 seconds, which correlates with an increase in oxygen production as shown by the sensor. According to the calculations made, 5.09 micromoles of oxygen were generated, corresponding to a FE of 94.1% for the oxygen evolution.

**Example 3. Effect of ligand and positively charged counterion on oxygen production (OER)**

Electrodes were prepared as described in example 2 of the present specification with the following compounds:

- Compound 1, as described in the present specification example 1; electrode 1 / CNT;
- Compound (TRIS)$_6$[V$_{10}$O$_{28}$], as the preparation of compound 2 is described in MISSINA, J.M., et al., Accessing decavanadate chemistry with tris(hydroxymethyl)aminomethane, and evaluation of methylene blue bleaching, Polyhedron, 01/02/2020, Vol. 180, pages 114414; electrode (TRIS)$_6$[V$_{10}$O$_{28}$]/CNT;
- Sodium vanadate; electrode Na$_6$[V$_{10}$O$_{28}$]/CNT

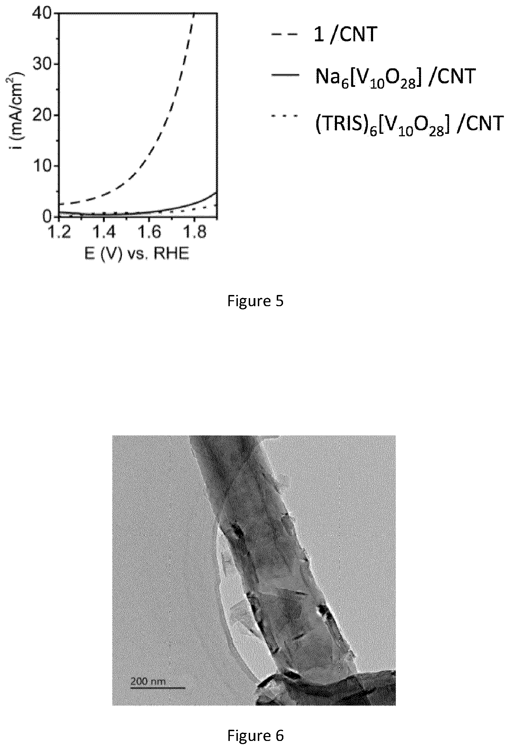

And the current density produced in the same potential range was compared and voltammograms were obtained for each of them.
It was observed that in the case of the electrode prepared with the compound obtained in example 1 of the patent application, when positive potentials slightly above 1.2 volts are applied, the electrode (1/CNT) is able to catalyse the oxidation of water producing oxygen, with the current density (mA/cm$^2$) obtained being proportional to the amount of oxygen produced. On the contrary, in the case of the other two electrodes prepared, it is necessary to apply much higher potentials to produce the same current density. Thus, when a potential of 1.8 volts is applied, the amount of oxygen produced by the compound obtained in example 1 is 20 times higher than that produced by the other two electrodes (Figure 5).
It is also demonstrated that the simultaneous presence of sodium and tris in the same compound is indispensable to obtain electrodes with higher electrocatalytic activity for the oxidation of water (oxygen production) at a lower applied potential.

**Example 4. Preparation of an electrode for hydrogen production (HER)**

4.1. Preparation of 1@CNT.

100 mg of **1** were dissolved in 1.5 ml of ionised water and added to a suspension of short CNT (5 mg in 1.5 ml of acetone) dropwise under sonication. The mixture was stirred at room temperature for 3 days and then filtered through a polytetrafluoroethylene (PTFE) membrane filter, washed repeatedly and with plenty of water to collect a yellowish black solid (20 mg). By high-resolution transmission electron microscopy (HR-TEM) it is observed that an amorphous material is assembled on the surface of the CNT, both on the external and internal side (Figure 6). By energy dispersive X-ray spectroscopy using STEM-EDS, which allows an elemental mapping of the sample, it is shown that the amorphous material is composed of vanadium, nitrogen and sodium, and therefore, vanadate, sodium ion and TRIS ion are deposited on the surface of the CNT.

4.2. Preparation of the HER electrode.

10 mg of the material (1 @ CNT) were carefully dispersed in 2 mL isopropanol with 40 $\mu$L of Nafion solution (1%) and sonicated for 15 min. Then, 200 $\mu$L of the suspension were deposited dropwise onto the glassy carbon electrode to form a film, with the final load being 1 mg of the material on the GCE (surface area =1 cm$^2$). The film thickness was optimised by adjusting the volume and/or the concentration of the graphene suspension. The electrode was allowed to dry before electrochemical measurements.

The same procedure was carried out with BC replacing the carbon nanotubes (CNT).

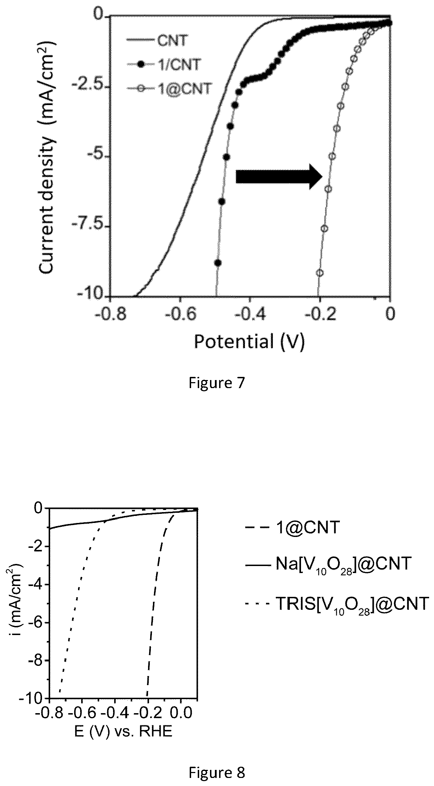

The change in the structure between compound 1 and the CNT obtained in example 4.1. versus that obtained in example 2.1. led to a change in electrochemical behaviour. In this way, the electrode prepared in 4.2. no longer showed activity in oxygen production and showed activity in hydrogen production (Figure 7).

Table 2. Comparative summary of the performance of different electrocatalyst materials for POM-based HER.

| Material | Electrolyte | Onset potential (mV) | Overpotential (mV) at -10 (mA/cm$^2$) | Tafel slope (mV/dec) | Reference |
|---|---|---|---|---|---|
| 1@CNT | 1M H$_2$SO$_4$ | -0.7 | 207 | 111 | Invention |
| h_MoN@BNCNT | 0.5M H$_2$SO$_4$ | -50 | | 46 | [1] |
| P$_2$W$_{18}$@rGF_ox | 0.5M H$_2$SO$_4$ | - | 35 | 87 | [2] |
| NENU-500 | 0.5M H$_2$SO$_4$ | -100 | 237 | 96 | [3] |
| V$_{10}$O$_{24}$.nH$_2$O | 0.5M H$_2$SO$_4$ | -0.1 | 118 | 101 | [4] |

Compared to other electrodes for hydrogen production, the electrode prepared in this example showed an excellent performance (Table 2).

[1] J. Miao, Z. Lang, X. Zhang, W. Kong, O. Peng, Y. Yang, S. Wang, J. Cheng, T. He, A. Amini, Q. Wu, Z. Zheng, Z. Tang, and C. Cheng, Adv. Funct. Mater., 2019, 29, 1805893.
[2] D. M. Fernandes, M. P. Araújo, A. Haider, A. Mougharbel, A. J. S. Fernandes, U. Kortz, and C. Freire, ChemElectroChem., 2018, 5, 273-283.
[3] J-S. Qin, D-Y. Du, W. Guan, X-J Bo, Ya-F. Li, Li-P. Guo, Z-M. Su, Y-Y. Wang, Y-Q. Lan, and H-C. Zhou, J. Am. Chem. Soc., 2015, 137, 7169-7177.
[4] K. K. Dey, S. Jha, A. Kumar, G. Gupta, A. K. Srivastava, and P. P. Ingole, Electrochem. Acta., 2019, 312, 89-99.

**Example 5. Effect of ligand and positively charged counterion on hydrogen production (HER)**

Electrodes were prepared as described in example 3 of the present specification with the following compounds:

- Compound 1 as described in the present specification example 1; electrode 1 @CNT;
- Compound (TRIS)$_6$[V$_{10}$O$_{28}$], as the preparation of compound 2 is described in MISSINA, J.M., et al., Accessing decavanadate chemistry with tris(hydroxymethyl)aminomethane, and evaluation of methylene blue bleaching, Polyhedron, 01/02/2020, Vol. 180, pages 114414; electrode (TRIS)$_6$[V$_{10}$O$_{28}$]@CNT;
- Sodium vanadate; electrode Na$_6$[V$_{10}$O$_{28}$]@CNT

And voltammograms were obtained for each of them.

It was observed that in the case of the electrode prepared with the compound obtained in example 1 of the present specification, when negative potentials below 0 volts are applied, the electrode (1@CNT) is able to catalyse the reduction of water producing hydrogen, with the current density (mA/cm$^2$) obtained in absolute value being proportional to the amount of hydrogen produced. On the contrary, in the case of the other two electrodes prepared, it is necessary to apply much higher and negative potentials to produce the same current density. Thus, when a potential of negative 0.2 volts is applied, the amount of hydrogen produced by the compound obtained in example 1 is more than 50 times greater than that produced by the other two electrodes, with the amount produced by $(TRIS)_6[V_{10}O_{28}]$@CNT being negligible at that potential (Figure 8).

It is also demonstrated that both the presence of sodium and the presence of tris are necessary to obtain electrode compounds with higher electrocatalytic activity for the reduction of water (hydrogen production) at a lower applied potential.

**Example 6. Battery at strong acidic pH**

To evaluate the behaviour of the electrocatalysts in the context of water splitting, a typical water splitting cell (two-electrode system) was built using 1/CNT at the positive electrode and 1@CNT at the negative electrode. The chronoamperometry test was carried out in a 1 M $H_2SO_4$ solution applying a potential of 3.1 V for 120 s. This behaviour was compared with the behaviour shown by the reference standards for Pt/C // Ir/C water splitting carried out under the same conditions, applying a voltage of 1.8 V.

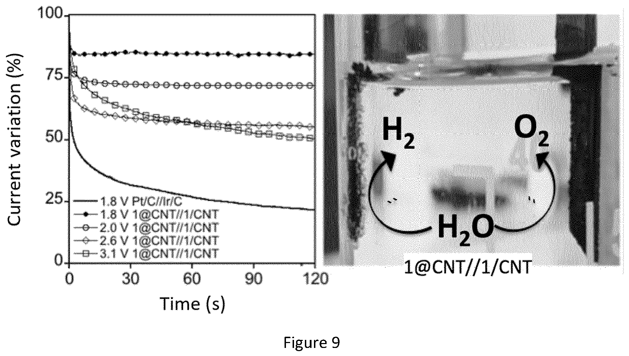

As shown in Figure 9, the configuration (1 @ CNT // 1 / CNT) under strong pH conditions (pH < 0.5) exhibited a much higher stability than that observed for the battery formed with the standard reference electrodes (Pt / C and Ir / C) under the same conditions. After 120 seconds, the drop in current density observed at 1.8 V for our electrolyser (15%) is more than five times lower than that observed for Pt / C // Ir / C (80%) at the same potential. However, for our battery, a higher potential (1.8 V vs. > 3 V) is required to initially produce the same current.

**Claims**

1. Compound (I) comprising

   a) a polyoxometalate,
   b) at least one ligand comprising a head which is capable of attracting protons and a tail which in an acidic medium has at least one positive charge,
   c) a positively charged counterion, wherein the positively charged counterion is selected from sodium, potassium and lithium,
   d) at least one water molecule.

2. Compound (I) according to claim 2, wherein the polyoxymethalate metal is selected from vanadium, tungsten, molybdenum, niobium, tantalum, zirconium and titanium.

3. Compound (I) according to any one of the previous claims, wherein the ligand head is a linear or branched, cyclic or non-cyclic hydrocarbon chain having between 1 to 12 carbon atoms, with more than 2 hydroxyl groups.

4. Compound (I) according to claim 3, wherein the ligand head is a group of formula $(AlqOH)_3Alq$, wherein each Alq can be the same or different and is independently selected from a linear or branched, cyclic or non-cyclic hydrocarbon chain having between 1 to 12 carbon atoms.

5. Compound (I) according to claim 3, wherein each Alq can be the same or different and is independently selected from a linear or branched non-cyclic hydrocarbon chain having between 1 to 4 carbon atoms.

6. Compound (I) according to any one of the previous claims, wherein the tail is selected from an amino group and phosphonium.

7. Compound (I) according to any one of the previous claims, wherein the ligand has the formula tris(hydroxy-alkyl($C_1$-$C_4$)aminoalkyl($C_1$-$C_4$).

8. Compound (I) according to claim 1, of formula $Z_n(H_2O)_m[(AlqOH)_3AlqNH_2]_l[M_XO_Y]$ wherein Z is an alkali metal selected from sodium, potassium and lithium,

   each Alq can be the same or different and is independently selected from a linear or branched, cyclic or non-cyclic hydrocarbon chain having between 1 to 12 carbon atoms,
   M is a transition metal selected from vanadium, tungsten, molybdenum, niobium, tantalum, zirconium and titanium,
   n has a value between 2 and 10

m has a value between 0 and 16,
l has a value of 1, 2, 3 or 4,
x has a value between 6 and 36,
y has a value between 16 and 112.

9. The compound of formula (I) according to claim 1, is $Na_4 (H_2O)_{12}[(CH_2OH)_3CNH_3]_2[V_{10}O_{28}].4H_2O$.

10. Electrode comprising the compound defined in any one of claims 1-9.

11. Electrode comprising a physical mixture of the compound (I) defined in any one of claims 1-9 and a carbon source.

12. Electrode comprising the compound (I), defined in any one of claims 1-9, assembled on the surface of a carbon surface.

13. A method for preparing an electrode according to claim 11, comprising:

a) grinding a carbon source,
b) mixing the result of step a) with the compound according to any one of the claims 1-10 until a homogeneous mixture is obtained, and then suspending it in an oil, and
c) spreading the suspension resulting from step b) on a support.

14. A method for preparing an electrode according to claim 12, comprising:

a) dissolving the compound according to any one of claims 1-9 in water,
b) adding the solution of a) under sonication onto a carbon source, previously ground,
c) filtering, and
d) resuspending the solid obtained in c), and
e) dispersing the mixture obtained in d) on a support.

15. Use of the electrode according to claim 11, for the production of oxygen or for the oxidation of water.

16. Use of the electrode according to claim 12, for the production of hydrogen or for the reduction of water.

17. Battery comprising an electrode according to any one of claims 10-12.

18. Battery according to claim 17, wherein the electrolyte has an acidic pH.

Figure 1

Figure 2

Figure 3

Figure 4

Figure 5

Figure 6

Figure 7

Figure 8

Figure 9

# INTERNATIONAL SEARCH REPORT

| International application No. |
| --- |
| PCT/ES2021/070649 |

### A. CLASSIFICATION OF SUBJECT MATTER

**See extra sheet**

According to International Patent Classification (IPC) or to both national classification and IPC

### B. FIELDS SEARCHED

Minimum documentation searched (classification system followed by classification symbols)
H01M, B01J, C01G, C25B

Documentation searched other than minimum documentation to the extent that such documents are included in the fields searched

Electronic data base consulted during the international search (name of data base and, where practicable, search terms used)

EPODOC, INVENES, WPI, NPL, XPESP, EMBASE, REGISTRY, CAPLUS

### C. DOCUMENTS CONSIDERED TO BE RELEVANT

| Category* | Citation of document, with indication, where appropriate, of the relevant passages | Relevant to claim No. |
| --- | --- | --- |
| A | MISSINA, J.M., et al., Accessing decavanadate chemistry with tris(hydroxymethyl)aminomethane, and evaluation of methylene blue bleaching, Polyhedron, 01/02/2020, Vol. 180, pág. 114414. Abstract and paragraph: "Experimental". | 1-18 |
| A | XU, W.-T., et al., Synthesis, Crystal Structure and Properties of a Novel Decavanadate Complex with One-dimensional Chain Involving Sodium and 'Bis-tris' Ligand, Chinese J. Struct. Chem, 31/03/2012, Vol. 31, pág. 401 407. Abstract and paragraph: "Experimental". | 1-18 |
| A | BAZZAN, I., et al., Cobalt based water oxidation catalysis with photogenerated Ru(bpy)33:Different kinetics and competent species starting from a molecular polyoxometalate and metal oxide nanoparticles capped with a bisphosphonate alendronate pendant, Catalysis Today, 10/04/2017, Vol. 290, pág. 39-50, ISSN 0920-5861, <DOI: doi:10.1016/j.cattod.2017.03.027>. Abstract. | 1-18 |

☒ Further documents are listed in the continuation of Box C.          ☐ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance. | | |
| "E" | earlier document but published on or after the international filing date | | |
| "L" | document which may throw doubts on priority claim(s) or which is cited to establish the publication date of another citation or other special reason (as specified) | "X" | document of particular relevance; the claimed invention cannot be considered novel or cannot be considered to involve an inventive step when the document is taken alone |
| "O" | document referring to an oral disclosure use, exhibition, or other means. | "Y" | document of particular relevance; the claimed invention cannot be considered to involve an inventive step when the document is combined with one or more other documents , such combination being obvious to a person skilled in the art |
| "P" | document published prior to the international filing date but later than the priority date claimed | | |
| | | "&" | document member of the same patent family |

| Date of the actual completion of the international search | Date of mailing of the international search report |
| --- | --- |
| 29/10/2021 | **(29/10/2021)** |

| Name and mailing address of the ISA/ <br><br> OFICINA ESPAÑOLA DE PATENTES Y MARCAS <br> Paseo de la Castellana, 75 - 28071 Madrid (España) <br> Facsimile No.: 91 349 53 04 | Authorized officer <br> M. García Poza <br><br><br> Telephone No. 91 3495568 |
| --- | --- |

Form PCT/ISA/210 (second sheet) (January 2015)

## INTERNATIONAL SEARCH REPORT

International application No.

PCT/ES2021/070649

C (continuation). DOCUMENTS CONSIDERED TO BE RELEVANT

| Category * | Citation of documents, with indication, where appropriate, of the relevant passages | Relevant to claim No. |
|---|---|---|
| A | CRANS, D.C., et al., Vanadium (V) complexes of polydentate amino alcohols: Fine-tuning complex properties, Journal of the American Chemical Society, 19/08/1998, Vol. 120, pág. 8069 - 8078, ISSN 0002-7863 (print), <DOI: doi: 10.1021/ja9808200>. Abstract. | 1-18 |
| A | TRACEY, A.L., et al., Vanadium (V) Oxyanions: Interactions of Vanadate with l,l,l-Tris(hydroxymethyl)ethane and with the Buffer Tris(hydroxymethyl)aminomethane, Inorg.Chem.1988, Vol. 27, pág. 1269-1275. Abstract. | 1-18 |

Form PCT/ISA/210 (continuation of second sheet) (January 2015)

## INTERNATIONAL SEARCH REPORT

International application No.

PCT/ES2021/070649

### CLASSIFICATION OF SUBJECT MATTER

*H01M4/58* (2010.01)
*B01J23/00* (2006.01)
*C01G31/02* (2006.01)
*C25B1/04* (2021.01)

Form PCT/ISA/210 (extra sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **Y. DING ; H. LI ; Y. HOU.** *Mater. Lett.,* 2018, vol. 221, 264-266
- **Y. WANG ; Y. WANG ; L. ZHANG ; C.-S. LIU ; H. PANG.** *Chem. Asian J.,* 2019, vol. 14, 2790-2795
- **M. MARTÍN-SABI ; J. SORIANO-LÓPEZ ; R. S. WINTER ; J-J. CHEN ; L. VILÀ-NADAL ; D-L. LONG ; J. R. GALÁN-MASCARÓS ; L. CRONIN.** *Nat. Catal.,* 2018, vol. 1, 208-213
- **G. Y. LEE ; I. KIM ; J. LIM ; M. Y. YANG ; D. S. CHOI ; Y. GU ; Y. OH ; S. H. KANG ; Y. S. NAM ; S. O. KIM.** *J. Mater. Chem. A.,* 2017, vol. 5, 1941-1947
- **MISSINA, J.M. et al.** Accessing decavanadate chemistry with tris(hydroxymethyl)aminomethane, and evaluation of methylene blue bleaching. *Polyhedron,* 01 February 2020, vol. 180, 114414
- **J. MIAO ; Z. LANG ; X. ZHANG ; W. KONG ; O. PENG ; Y. YANG ; S. WANG ; J. CHENG ; T. HE ; A. AMINI.** *Adv. Funct. Mater.,* 2019, vol. 29, 1805893
- **D. M. FERNANDES ; M. P. ARAÚJO ; A. HAIDER ; A. MOUGHARBEL ; A. J. S. FERNANDES ; U. KORTZ ; C. FREIRE.** *ChemElectroChem,* 2018, vol. 5, 273-283
- **J-S. QIN ; D-Y. DU ; W. GUAN ; X-J BO ; YA-F. LI ; LI-P. GUO ; Z-M. SU ; Y-Y. WANG ; Y-Q. LAN ; H-C. ZHOU.** *J. Am. Chem. Soc.,* 2015, vol. 137, 7169-7177
- **K. K. DEY ; S. JHA ; A. KUMAR ; G. GUPTA ; A. K. SRIVASTAVA ; P. P. INGOLE.** *Electrochem. Acta.,* 2019, vol. 312, 89-99